# EUROPEAN PATENT SPECIFICATION

(11) **EP 3 099 818 B1**
(45) Date of publication and mention of the grant of the patent: **18.03.2020**
(21) Application number: 15707424.6
(22) Date of filing: 30.01.2015
(51) Int. Cl.: C12Q 1/6883

(54) **PREIMPLANTATION ASSESSMENT OF EMBRYOS THROUGH DETECTION OF FREE EMBRYONIC DNA**
PRÄIMPLANTATIONSBEURTEILUNG VON EMBRYONEN DURCH ERKENNUNG VON FREIEM EMBRYONALEN DNA
ÉVALUATION PRÉIMPLANTATOIRE D'EMBRYONS PAR DÉTECTION DE L'ADN EMBRYONNAIRE LIBRE

(30) Priority: 30.01.2014 US 201461933340 P; 10.10.2014 US 201462062182 P
(43) Date of publication of application: 07.12.2016
(73) Proprietor: Pécsi Tudományegyetem, 7622 Pécs (HU); Bay Zoltán Alkalmazott Kutatási Közhasznú Nonprofit KFT., 1116 Budapest (HU)
(72) Inventor: BÓDIS, József, H-7635 Pécs (HU); KOVÁCS, L. Gábor, H-7635 Pécs (HU); VERMES, István, H-7621 Pécs (HU); FEKETE, Csaba, H-7624 Pécs (HU); RIDEG, Orsolya, H-7622 Pécs (HU); BIHARI, Zoltán Endre, H-6726 Szeged (HU); PACH, Ferenc Péter, H-8175 Balatonfüzfö (HU); BATÓ, Emese, H-6723 Szeged (HU); PAPP, Ildikó, H-6600 Szentes (HU); GÁLIK, Bence, H-5920 Csornás (HU); SZERZÖ, Csaba, H-5123 Jászárokszállás (HU)
(74) Representative: Lengyel, Zsolt
(86) International application number: PCT/IB2015/050701
(87) International publication number: WO 2015/114574

(56) References cited:
- WO-A1-2013/056002
- WO-A1-2013/116889
- WO-A1-2014/116881
- WO-A1-2014/202696
- FUMAN JIANG ET AL: "Noninvasive Fetal Trisomy (NIFTY) test: an advanced noninvasive prenatal diagnosis methodology for fetal autosomal and sex chromosomal aneuploidies", BMC MEDICAL GENOMICS, BIOMED CENTRAL LTD, LONDON UK, vol. 5, no. 1, 1 December 2012 (2012-12-01), page 57, XP021137777, ISSN: 1755-8794, DOI: 10.1186/1755-8794-5-57
- S. STIGLIANI ET AL: "Mitochondrial DNA content in embryo culture medium is significantly associated with human embryo fragmentation", HUMAN REPRODUCTION, vol. 28, no. 10, 1 October 2013 (2013-10-01), pages 2652-2660, XP055191627, ISSN: 0268-1161, DOI: 10.1093/humrep/det314
- SIMPSON J L: "I1 Clinical challenges in application of prenatal technologies", REPRODUCTIVE BIOMEDICINE ONLINE, REPRODUCTIVE HEALTHCARE LTD, GB, vol. 26, 12 May 2013 (2013-05-12), XP028539797, ISSN: 1472-6483, DOI: 10.1016/S1472-6483(13)00237-X
- ASSOU SAID ET AL: "Non-invasive pre-implantation genetic diagnosis of X-linked disorders", MEDICAL HYPOTHESES, vol. 83, no. 4, October 2014 (2014-10), pages 506-508, XP029057355, ISSN: 0306-9877, DOI: 10.1016/J.MEHY.2014.08.019

## Description

According to data of GeoHive, till September of 2014, population size of the world was around 7,2 billion and this number is increasing (www.geohive.com), the average growth rate is around 1,10% annually.

In contrast, developed countries show negative demographic tendencies. Despite international calls for the prevention and appropriate treatment of infertility, this condition is becoming more common throughout the developed world. The decline in the overall health standard of the population (obesity, escalating incidence of sexually transmitted diseases that affect reproductive organs) can, at least partially, help to explain the increase in infertility. Furthermore the postponement of having the first child is also becoming more and more common. Together, these health and social considerations mean that the number of infertility cases is increasing.

Since the first groundbreaking work on in vitro fertilization (IVF), assisted reproductive technology (ART) has made considerable progress to overcome human sub- and infertility (Gnoth et al., 2005; Habbema et al., 2009). Nowadays 3-4% of total births are concepted by assisted reproductive procedures. Despite some astonishing theoretical and technological advances achieved by ART (Wang and Sauer, 2006), the practical implementation of "healthy baby" concept still pose unique challenges (Arce et al., 2013). The success rate of the procedure is only around 30-34%. Due to the evidence that transferring multiple embryos increase the risk for adverse perinatal outcomes (Hansen et al., 2005.), improving reliability of implantation-competent embryos is increasingly emphasized in clinical practice. Difficulties encountered in selecting the most viable embryos are strongly associated both with the inconsistency of results obtained by different biomarkers and limited knowledge of multifactorial background of embryonic development.

It is widely accepted that programmed cell death plays a significant role in early stage of embryogenesis, nevertheless, the link among apoptosis, degree of embryo- and DNA-fragmentation has not yet been definitively elucidated (Fujimoto et al., 2011).

In the current clinical practice of ART, embryos are selected for transfer largely based on morphological evaluation. Even though, there is a strong correlation between embryo morphology and implantation rate (Vernon et al., 2011), visual assessment of embryo quality remains problematic in terms of (I.) number and inter related properties (hierarchy) of prognostic variables; (II.) unknown consequences of dysmorphic phenotypes; (III) discontinuous observation of a dynamic process; (IV.) limitations of traditional microscopy extracting quantitative data from images; (V.) variety of the existing scoring systems and also their biases could dramatically affect the comparability and reliability of the prediction (Balaban et al., 2011; Machtinger and Racowsky, 2013).

To overcome the limitations and discrepancies derived from morphological grading, developing alternative molecular based non-invasive methods are in the focus of interest.

Methodological advances and innovation in existing techniques as fluorescence in-situ hybridization (FISH), real time PCR and array-based comparative genomic hybridization (aCGH) have the potential to make an important contribution to qualitative and quantitative research. Next generation sequencing (NGS) based approaches (as massively parallel shotgun sequencing (MPSS), targeted sequencing, SNP sequencing) provide exceptional opportunity to identify whole chromosome's structural and numerical abnormalities.

Many areas of biology have a long tradition in utilizing molecular markers such as metabolic parameters, secreted proteins, nevertheless, the landmark discovery of cell-free DNA opened up new tools for non-invasive molecular diagnostics.

Prognostic value of cell free DNA has been reported in various disease conditions (Holdenrieder and Stieber, 2009), and also as a part of non-invasive prenatal testing (NIPT). Nevertheless the implementation of cfDNA-based diagnosis is still a matter of debate, largely due to the lack of gold standard in DNA extraction, and in characterization of fragmented DNA. Biological artifacts like non-template nucleotide addition, microvariants, tri-allelic patterns, nullallels, allelic drop out could lead to incorrect genotyping of the individual.

Several lines of evidence suggest that genetic profiling of spent embryo culture media should offer exceptional opportunity for non-invasive assessment of embryo viability (Stigliani et al., 2013).

It has been well established that chromosome abnormalities contribute significantly to genetic diseases resulting in reproductive loss, infertility, stillbirths, abnormal sexual development. They are present in at least 50% of spontaneous abortions, 6% of stillbirths, about 5% of couples with two or more miscarriages and approximately 0.5% of newborns. In women aged 35 or over, chromosome abnormalities are detected an about 2% of all pregnancies. Couples participating in ART processes are more often affected by known or latent numerical and/or structural chromosomal abnormalities, and there is a significant increase in de novo sex and autosomal chromosome aberrations after ART (Devroey et al, 2004).

The most common clinically significant chromosomal aneuploidies are defined as an abnormal number of chromosomes and may involve the autosomal or sex chromosomes. The majority of chromosomal aneuploidies are non-viable, leading to early miscarriage. However, a subset may survive to the new born period and beyond, including trisomy 21 (Down syndrome), trisomy 18 (Edwards syndrome), and trisomy 13 (Patau syndrome). Together, these occur in approximately 1 in 450 live births). When including sex chromosome aneuploidies, such as monosomy X (Turner syndrome), this increases to approximately 1 in 250 live births. Overall, trisomy 21 is the most prevalent at-birth aneuploidy, and the most common congenital cause of mental retardation (Gardner et al., 2012). Other aneuploidies, including trisomies 13 and 18, are associated with significant clinical morbidity and a high neonatal mortality rate. Thus, identification of such fetuses early in pregnancy would provide mothers/parents with sufficient time to consider reproductive options. Numerical chromosomal imbalances are often (not always) linked to structural chromosomal abnormalities as balanced (Robertsonian) and unbalanced rearrangements. Unbalanced carriers are always associated with clinical abnormalities owing to their inherent genetic imbalance. While patients who are balanced carriers have a dramatically elevated risk of producing gametes with an incorrect number of chromosomes.

First clinical use of prenatal genetic testing in the early 1990s was applied to determine fetal sex using Y-chromosome specific PCR-based assays (Handyside et al., 1990). Fetal sex determination is offered for carriers being at high risk of transmitting X - linked recessive disorder (as Hemophilia A; B, Duchenne or Becker muscular dystrophy, Red-green color blindness etc) and for carriers of Y-linked abnormalities (as AZF deletions, retinitis pigmentosa, color blindness, XYY syndrome). In such cases preimplantation sex determination provides the opportunity for transferring healthy female embryo.

WO2013/116889 describes theoretical discussion on fetal cell free nucleic acid analysis and suggests FISH to carry out the detection.

WO2014/116881 discloses the determination of genomic copy alteration from RNA isolated from vesicles present in the culture medium of embryos.

Assau et al (Medical hypotheses, vol. 83 no. 4, pp 506-508, 2014) discloses that Y-linked genes may be detected in the culture medium of male embryos but not in female embryos.

WO2014/202696 discloses that nucleic amplification of cell free DNA can be used for sex determination, especially mentioning Y chromosome linked genes TSPY1 (Testis specific protein, Y linked 1) and RPS4Y1 (Ribosomal protein S4, Y-linked 1).

Apoptosis is characterized by a set of morphological and biochemical events, among which appearance of high molecular weight, and oligonucleosomal DNA fragmentation appears.

To avoid concluding false genetic information from the embryo due to poor quality of the cell free fetal DNA, quality assessment must be take into consideration.

Based on observations in forensic science short tandem repeat (STR) analysis provides capable technology for degraded DNA quality assessment. Short Tandem Repeats (STR), also referred to as microsatellites, are tandem repeated DNA sequences widespread throughout the human genome. The number of repeats can vary from 3 to more than 50 repeats with extremely polymorphic structure, making them important genetic markers for human identity testing. Repeating units are 2-6 bp in length, and are readily amplified during PCR by using primers that bind in conserved regions of the genome flanking the repeat region. Detection of multiple makers (13-16 STR markers) at a time provides genetic fingerprint of the person. STR markers provide considerable advantages over previously used methods for DNA fingerprinting including the ability to obtain results from small amounts and degraded DNA samples.

Currently, the standard of care for pregnant women in developed countries involves discussion of the various prenatal diagnosis options, including non-invasive screening and invasive diagnostic methods. Therefore there is still a need in the art to provide a non-invasive, highly accurate test to increase the success rates of ART.

The present disclosure is aimed to provide accurate and reproducible procedures for preimplantation genetic diagnosis, analyzing cell free nucleic acids (DNA, RNA, miRNA) released by the embryo into the culture medium on the 3rd (8-10 cells) and 5th (blastocyst) days of incubation. The assessment of embryos is enabled by the detection of cell free DNA content of 3rd and 5th days spent embryonic medium using targeted TaqMan PCR assay. In specific embodiments, prevalence of Y-chromosome in male embryos' spent medium was proved by applying Y-chromosome identical, DYS14 gene targeted genetic analysis. According to the results, the cell free nucleic acid content of embryos' spent medium provides a novel approach for non-invasive preimplantation genetic testing.

In another aspect, the description discloses employing the available detection technologies for identifying STRs, cell free nucleic acid content of embryos' spent medium may also be assessed. Considering that STR detection technology allows for the detection and typing of low amounts and degraded DNA, the STR results, such as electropherograms could provide information from the embryonic culture medium samples's quality. In a further aspect, aneuploidy of the embryo may be assessed through applying full genome sequencing techniques. It is indeed a very surpising finding that such an analysis of the cell free DNA fragments of the culture medium of a 3-5 days old embryo gives valid results that are directly correlate to the quality / later viability of the embryo.

Accordingly, the present invention provides an *in vitro* method for non-invasive preimplantation assessment of an embryo, comprising
a) providing a sample taken from the *in vitro* culture medium of the embryo on the 3^{rd} day of incubation;
b) submitting the sample to nucleic acid amplification with primers specific to a sequence of interest indicative of a genetic deficiency;
c) detecting the amplified nucleic acid by PCR,
wherein the presence of the nucleic acid amplified from the culture medium suggests the presence of the corresponding sequence in the embryo cultured in said medium, thereby enabling the preimplantation assessment of the embryo with respect of the sequence of interest,
wherein the embryo is assessed as having a genetic deficiency if the if the presence of the sequence of interest is detected in the *in vitro* culture medium of the embryo.

In an embodiment the nucleic amplification is real time PCR.

In another embodiment, the detection includes detecting fluorescent labels.

In a further embodiment, the sequence of interest is a gene and/or an allele thereof and/or a variant thereof carrying a specific mutation.

In another preferred embodiment, the gene is located on the Y chromosome.

In another embodiment, the gene is known to be involved in developmental disorders.

In a further aspect of the invention, the method involves carrying out linear amplification-based full genome sequencing, wherein the presence of aneuploidy is indicative of an embryo having a limited chance for resulting in successful pregnancy.

In a preferred embodiment, aneuploidy is evaluated by z-tests.

The disclosure of Stigliani et al. (2013) about nuclear- and mitochondrial DNA released by the embryo into the culture medium may be considered as the closest prior art. However, it is clear from the findings presented therein that no positive correlation between embryo score and DNA profiles can be established. There is no suggestion that specific genes or genetic deficiencies could be detected and/or identified based on the presence of cell free DNA obtained from the embryo culture media.

Embryos with abnormal chromosomes are thought to explain significant proportion of failed IVF cycles. However false negative-rates (12-23%) and false-positive rates (1.9-5.2%) of the currently used traditional non-invasive methods (as ultrasound and maternal serum markers) and invasive procedures (as amniocentesis or chorionic villus sampling) are high (Brynn Levy, 2013). This, coupled with the high risk of procedural miscarriage following invasive procedures (1 in 300 cases) propose the need of developing new accurate and reliable non-invasive genetic methods for the assessment of embryos' viability before maternal transfer.

In our study using beta globin gene identical assay we proved the prevalence of cell free DNA in 3 day and 5 day embryonic spent medium. After this verification, the prevalence of Y-chromosome in "male" 3 day and 5 day embryonic droplets using DYS14 identical assay were checked and confirmed. As a further proof of the present invention, identification of other autosomal genes/mutations was carried out. These specific examples are ones that might show a link to certain genetic diseases like cistic fibrosis / CFTR gene or genes/mutations that might tend to develop certain disease like prothrombin gene, leiden gene (thrombosis), multidrug resistance 1(mdr1) gene (therapy resistance). In our study we verified the prevalence of certain autosomal genes such as prothrombin, leiden and mdr 1 in 3 day and 5 day embryonic spent medium using gene identical SYBRgreen assay and melting curve analysis.

According to our results, genetic profiling of 3rd and 5th days embryonic spent media cell free nucleic acid content (DNA, RNA, miRNA) - could provide new preimplantation genetic screening opportunity to increase the success rates of ART. Disclosed is the use of STR profiling to characterize the cell free DNA of the embryonic cultures. Although the methodology was developed for forensic analysis of scarce nucleic acid samples, its usefulness is questionable on samples of embryonic cell free DNA. The state of the art has no information in this regard.

The present description establishes a clear disclosure that the standard forensic assay methodology is applicable on samples derived from standard ART procedures. As detailed in the examples, the STR profiles obtained from the ART samples is useful to provide clear differentiation between with good and bad quality DNA, therefore assisting in the decision whether further test may or should not be attempted.

In a third aspect, full genome sequencing may be used to analyze cell free DNA content of samples derived from embryonic culture media. In this aspect, although the cell free DNA is very fragmented, and no full genomic information may be obtained in the usual sense, the present inventors found that the samples analyzed using the protocols detailed herein give crucial information on the aneuploidy status of a sample. Based on the statistical evaluation of genome-wide NGS data, embryos carrying lethal aneuploidies can be filtered out during the pre-implantation period.

Both aspects of the present invention are in common in their ability to effectively forecasting whether a specific embryo is viable or not, i.e. has a good chance for continued development. It is apparent that the presence of genetic deficiencies may direct consequences on the later development and viability of the embryo. In this context of the present invention, the term "genetic deficiency" is defined as any modification in the genome that results in mutations that manifested in altered phenotype. Further, a "viable" embryo is defined as an embryo, which, after implantation, develops into a healthy fetus, and results in successful pregnancy. It must be noted that an embryo may still be "viable" if it contains one or more "genetic deficiency". However, when the practitioner has a choice between embryos with and without "genetic deficiency", the chance to implant a "viable" embryo is higher when selecting the one without genetic deficiency.

It is clear that both aspects of the present invention is used as a non-invasive method to assess embryo viability, using simple samples of culture mediums that are disposed after implanting the embryos grown therein. The procedures themselves are mostly automated and may be carried out within reasonable time periods, thus allowing the practitioners skilled in ART techniques to make informed decisions on the selection of the embryos, which give the best chance for successful pregnancy in the case of any given patient.

### Brief description of the drawings

A brief summary of each of the figures is provided below:

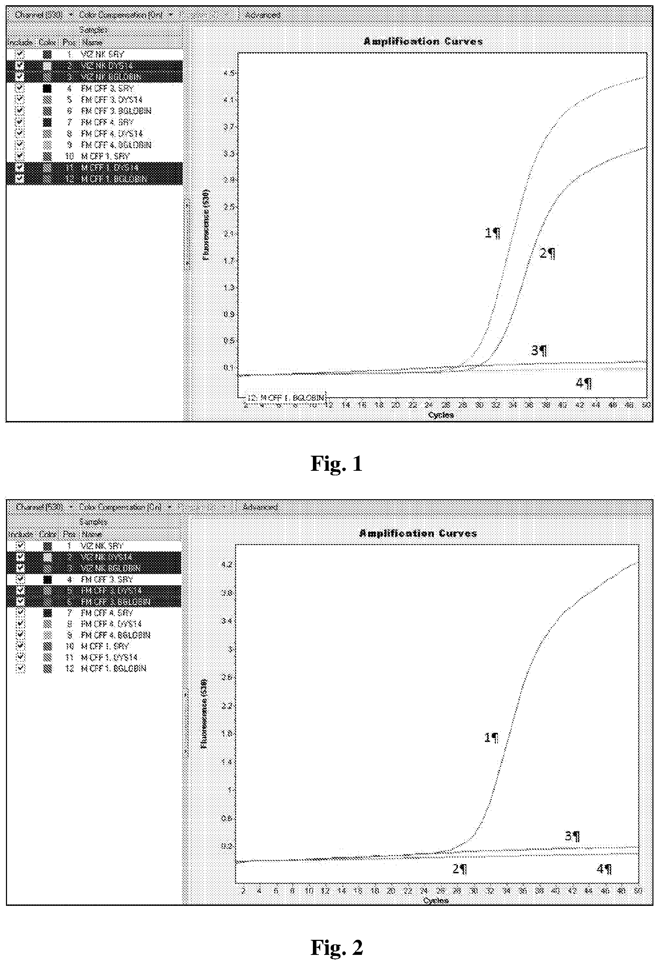

Figure 1 shows a graph of beta globin gene and DYS14 gene amplification in male embryonic culture media. Line 1 (brown): positive amplification curve of beta globin gene in male embryonic culture media; line 2 (blue): positive amplification curve of DYS14 gene in male embryonic culture media; line 3 (red): negative amplification curve of beta globin gene in negative control sample; line 4 (green): negative amplification curve of DYS14 gene in negative control sample.
Figure 2 shows a graph of beta globin gene and DYS14 gene amplification in female embryonic culture media. Line 1 (blue): positive amplification curve of beta globin gene in female embryonic culture media; line 2 (pink): negative amplification curve of DYS14 gene in female embryonic culture media; line 3 (red): negative amplification curve of beta globin gene in negative control sample; line 4 (green): negative amplification curve of DYS14 gene in negative control sample.

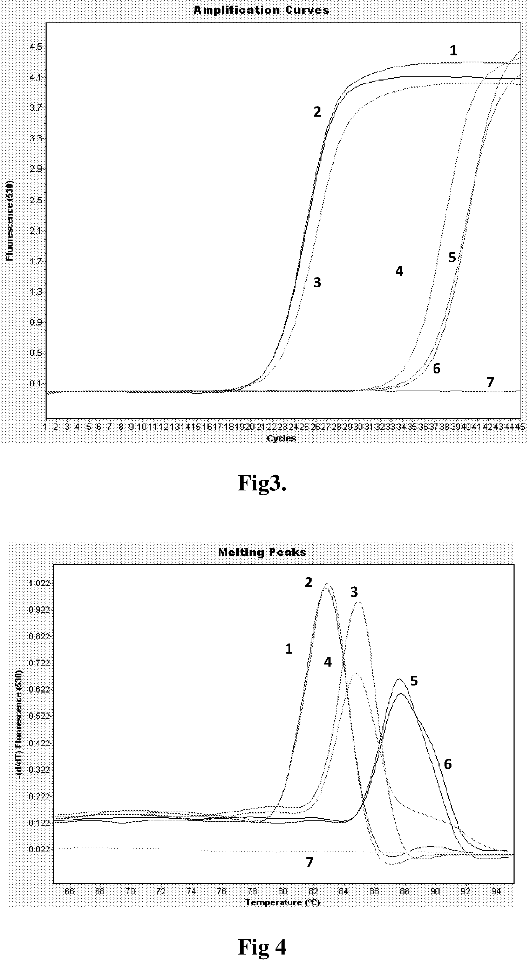

Figure 3 shows a graph of prothrombin gene, leiden gene and mdr 1 gene amplification in embryonic culture media. Line 1 (dark brown): positive amplification curve of leiden gene in human control; line 2 (gray): positive amplification curve of mdr 1 gene in human control; line 3 (golden brown): positive amplification curve of prothrombin gene in human control; line 4 (pink): positive amplification curve of mdr 1 gene in embryonic culture media; line 5 (light gray): positive amplification curve of prothrombin gene in embryonic culture media; line 6 (brown): positive amplification curve of leiden gene in embryonic culture media; line7 (purple): negative control.
Figure 4 shows a graph of prothrombin gene, leiden gene and mdr 1 gene melting curve analysis. Line 1 (dark brown): melting curve of leiden gene in human control; line 2 (light brown): melting curve of leiden gene in embryonic spent media; line 3 (blue): melting curve of prothrombin gene in human control; line 4 (golden brown): melting curve of prothrombin gene in embryonic spent media; line 5 (purple): melting curve of mdr 1 gene in human control; line 6 (black): melting curve of mdr 1 gene in embryonic spent media; line 7 (light blue): melting curve of negative control.

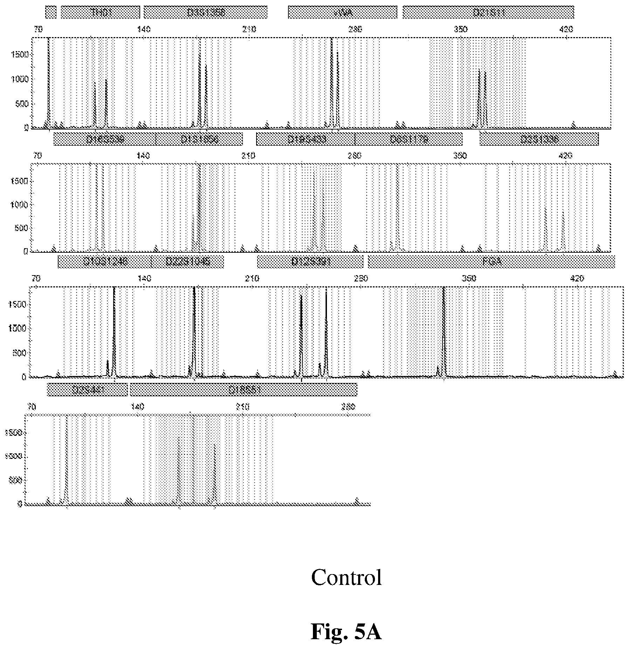

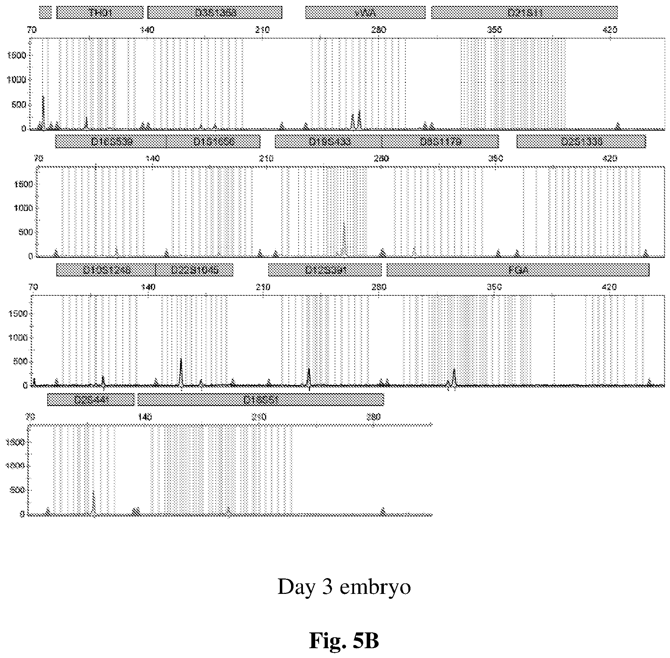

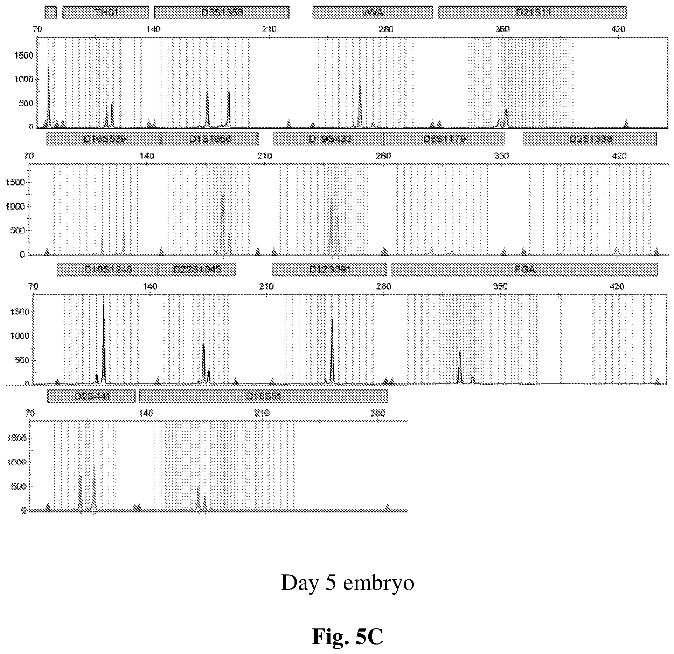

Figure 5 shows a representative electropherograms of Short tandem repeat (STR) profiling. Panel A, control sample of healthy Caucasian volunteer; panel B, electropherogram of day 3 embryo; panel C, electropherogram of day 5 embryo. Red triangles indicate the range of the certain chromosomal marker; gray lines indicate the places of already described alleles; pink lines indicate the places of hypothetical alleles.

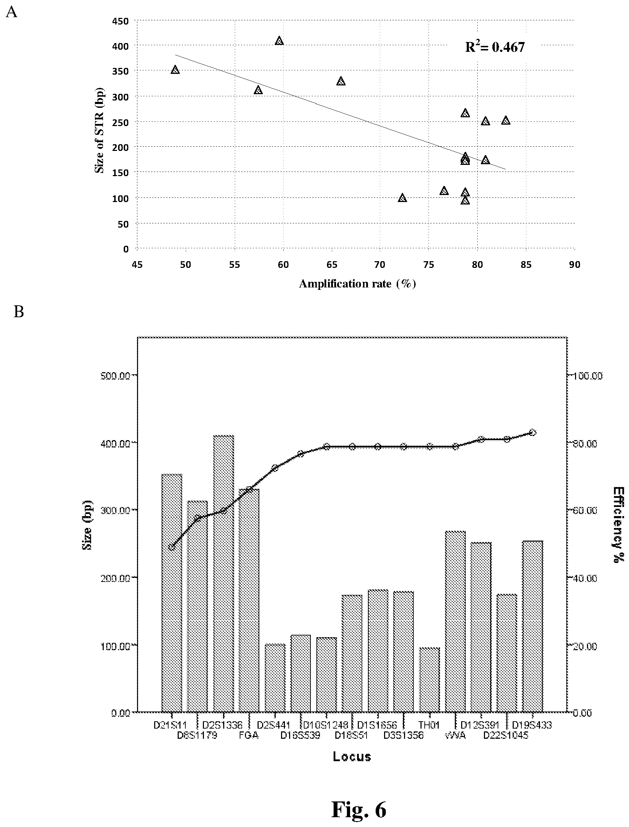

Figure 6, Panel A shows the correlation between size of STR markers and amplification rate; panel B, size and efficiency comparison of individual STR loci.

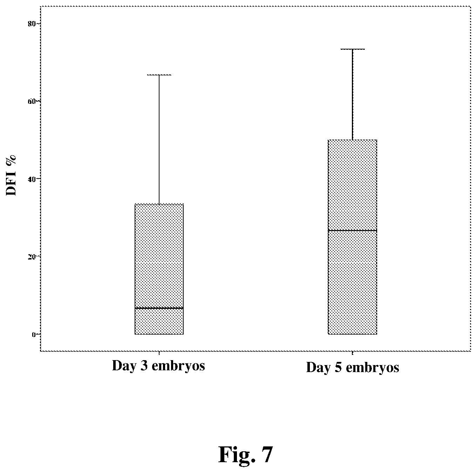

Figure 7 shows box plots reflecting the distribution of cftDNA integrity index (DFI) of day 3 and day 5 embryos. The horizontal line inside the box indicates the median.

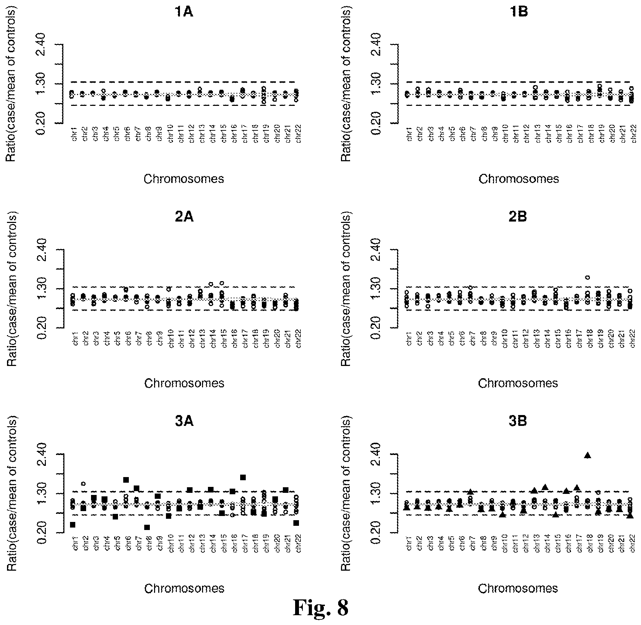

Figure 8 shows the statistically evaluated NGS data of the distinctive 3 different embryonic DNA types. Mapped autosome sequencing read ratios of individual IVF cases and the mean control autosomes are represented per chromosomes. Panels marked with A represent the 3-day, whilst with B correspond the 5-day embryonic state. 1: morphologically non-viable embryos; 2: unsuccessully implantable embryos; 3: successully implantable embryos. Grey lines represents the 95% confidence intervals of healthy volunteers, dashed lines refer to the artificial ratio thresholds of successful pregnancy. Black squares (3A) and triangles (3B) show the ratio values of successfully implanted, but later spontaneously aborted embryos.

### Methods and Materials

### Ethical approval

The study was reviewed and approved by the Ethics Committee of University of Pécs (Approval No.: 5273-2/2012/EHK). Signed informed consent was obtained from all patients who participated in the study. The investigation conforms to the principles outlined in the Declaration of Helsinki.

### Oocyte processing and embryo culture systems

Procedure of follicles aspiration was guided by Sono Ace 6000C ultrasound system (Samsung Medison). For the purpose of reducing potential pH, osmolality and thermal imbalances, pre-warmed G-MOPS™ medium (Vitrolife) was applied for handling of oocytes at ambient atmosphere. Denudation of individual oocytes was carried out enzymatically in diluted HYASE-10X™ (Vitrolife) according to the large volume method recommended in a G-series manual 6.0 (http://www.vitrolife.com). On the day of oocytes retrieval (Day 0), ICSI was performed on mature (metaphase II) oocytes according to the laboratory's routine insemination procedures (Bódis et al., 2010). The injected oocytes were individually cultured in human serum albumin (HSA) supplemented sequential medium G-1TMv5 and G-2TMv5 (Vitrolife), optimized for the processes of early embryogenesis during day 1-3 and day 3-5 respectively. Culture of human embryos in microdroplets of IVF medium were covered with mineral oil and incubated at 37.0°C in humidified three gas atmosphere of 6% CO2, 5% 02 and 89% NO2 using bench top incubator (MINC).

### Embryo quality assessment and sample collection

Normal fertilization was assessed microscopically 15 to 20 hours after ICSI (day 1) with the appearance of two pronuclei, polarbodies and available of a germinal vesicle. Grading of early embryos (day 3) were based on the actual number of (blastomeres) cells, degree of fragmentation and uniformity of stage specific cell size. On day 5, morphological assessment of blastocysts were scored according to combination of development and grade of the inner cell mass (ICM) and trophoectoderm (TE) as recommended by Alpha Scientists in the Istanbul consensus workshop on embryo assessment (Balaban et al., 2011). Morphological features of embryos were observed immediately prior to the scheduled transfer using a Diaphot 300 inverted phase contrast microscope (Nikon) equipped with image acquisition and archival software (OCTAX EyeWear™v1.5). The embryos identified for transfer, either on day 3 or day 5 depending on the stage of development were removed from the droplets with minimal volume of media and separated for implantation process. Consented individuals were undergoing double embryo transfer. The remaining 40 µl spent embryo culture media were collected, flash-frozen in liquid nitrogen and stored in -80 °C until DNA extraction.

### Outcomes measures

To determine success rates of ICSI treatments, clinical pregnancy were confirmed through ultrasonic observations of the intrauterine gestation sac. Numbers of live births/transfer and the gender of the fetuses were identified during and after birth. Despite of the double embryo implantation processes, 100% of the observed transfers resulted in single births.

### DNA Extraction and quantitation

To enhance cell free total DNA (cftDNA) recovery from low concentration spent embryo culture media QIAamp Min Elute Virus Spin Kit (Qiagen) was routinely used according to the manufacturer's recommendations. The final purified DNA was not diluted. Optionally, cftDNA can be captured by paramagnetic bead separation or gel filtration techniques.

All extracted cftDNA samples were initially analyzed with a NanoDrop 2000 spectrophotometer (ThermoScientific) and/or Qubit Fluorometer (Life Technologies), and then further assayed on an Agilent Technologies 2100 Bioanalyzer using High Sensitivity DNA kit and/or DNA 1000 Kit (Agilent Technologies).

Embryo-free microdroplets of sequential media (G-1 and G-2) incubated alongside the embryo-containing culture were used as a negative control in each experiment.

### Example 1 - Detection of single genes

### Sample selection

To avoid the discrepancies in gender identifying, turning up from double transfer - single live birth, droplets of transfer at a time were analyzed parallel. Spent culture media was signed as "male" embryo's environment if Y-chromosome was detected in both droplets and the gender of the newborn proved the result of the genetic analysis. Spent culture media was signed as "female" embryo's environment if Y-chromosome was not presented in either droplets or the gender of the newborn proved the result of the genetic analysis.

Based on the number of live births, in our retrospective study, out of 25 embryonic spent culture media 16 were signed as "male" droplet and 9 were signed as "female" droplet. Out of the 16 "male" media 11 were collected on day 3, 5 on day 5. Out of the 9 "female" media 5 were collected on day 3, 4 on day 5.

### Realtime PCR amplification

DNA from individual embryos and blank media controls were amplified with LightCycler 2.0 system (Roche Applied Biosystem). Ten-fold serial dilutions of control total human genomic DNA (Roche; Cat. N.:1 691112; 0.2µg/µl; 10⁴-10⁰) was used as beta globin standard curve. Ten-fold serial dilutions of DYS14 plasmid (10⁸ copies/µl; 10⁸-10⁰) were used as DYS14 gene standard curve.

The PCR amplification was performed with LightCycler TaqMan Master mix (Cat.N.: 04535286001). Ingredients: TaqMan PCR mix 5x: 4 µL; H₂O: 9 µL; Fwd/Rev primers (see Table 1): 0.6 µL (0.3 µM); Taqprobe: 0.8 µL (0.4 µM); DNA template: 5 µL. The total reaction volume was 20 µL. Quantitative real time PCR was performed under the following conditions: initial step of 95°C for 10 minutes, 50°C for 2 minutes followed by 50 cycles of 95 °C for 15 s, 68 °C for 1 minute 20 °C/s 50x; the reaction was terminated at 40°C for 30 s.

Results were analysed by LightCycler 4.05 software version.

**Table 1. Beta globin gene, DYS14 gene, Prothrombin gene, Leiden gene and Multidrug resistance gene primer and probe sequences and accession numbers.**

| **H. sapiens HBB hemoglobin, beta gene** | (**NG_000007**) |
|---|---|
| Forward primer | 5'-GTGCACCTGACTCCTGAGGAGA-3' |
| Reverse primer | 5'-CCTTGATACCAACCTGCCCAG-3' |
| TaqMan probe | 5-6FAM-AAGGTGAACGTGGATGAAGTTGGTGG-TMR-3' |

| **H. sapiens TSPY1 testis specific protein, Y-linked 1 (DYS14)** | **NG_027958** |
|---|---|
| Forward primer | 5'-GGGCCAATGTTGTATCCTTCTC-3' |
| Reverse primer | 5'-GCCCATCGGTCACTTACACTTC-3' |
| TaqMan probe | 5'-FAM-AGCACCTCTCCACTAGAAAGGCCG-BBQ-3' |

| **Prothrombin gene** | **NG_M17262** |
|---|---|
| Forward primer | 5'-CCGCTGGTATCAAATGGG-3' |
| Reverse primer | 5'-CCAGTAGTATTACTGGCTCTTCCTG-3' |

| **Leiden gene** | **NG_M17262** |
|---|---|
| Forward primer | 5'-TGCCCAGTGCTTAACAAGACCA-3' |
| Reverse primer | 5'-CTTGAAGGAAATGCCCCATTA-3' |

| **Multidrug resistance 1 gene** | **NG_5343** |
|---|---|
| Forward primer | 5'-TGTTTTCAGCTGCTTGATGG-3' |
| Reverse primer | 5'-AAGGCATGTATGTTGGCCTC-3' |

### Detection of Y-chromosomal gene

Individuals participating in our retrospective study were undergoing double embryo transfer, however 100% of the transfers resulted in single live births. To avoid the discrepancies in gender identifying, turning up from double transfer - single live birth, droplets of transfer at a time were analysed in parallel.

Based on the number of total live births, out of 25 embryonic spent culture media 16 were signed as "male" droplet and 9 were signed as "female" droplet. Out of the 16 "male" media 11 were collected on day 3, 5 on day 5. Out of the 9 "female" media 5 were collected on day 3, 4 on day 5. Every spent medium was screened for beta globin gene and DYS14 gene.

Beta globin gene was detected in every sample using a Ct value cutoff 35. The average Ct value was 24,1. None of the negative controls gave false positive result. See Fig. 1.

Out of the 16 "male" droplet samples of (see Fig. 1.) the DYS14 gene was detected in 11 cases, 6 from day 3, 5 from day 5, using the Ct value cutoff 35. The average Ct value was 31. Neither of the "female" droplets and the negative controls gave false positive result. See Fig. 2.

Out of the 16 "male" droplet samples DYS14 gene was detected in 11 cases, 6 from day 3, 5 from day 5. Based on the results and birth outcome in these cases boy embryo twins were transferred. Though in 5 cases we were not able to detect the DYS14 gene in either of the parallel tested mediums, based on the results and birth outcome in these cases either boy embryo twins or boy - girl twins were transferred.

Neither of the "female" droplets gave false positive DYS14 gene result.

### Detection of mdr 1 gene, prothrombin gene and leiden gene

### Sample selection

Based on the standardized DNA extraction method as described above, a total of 20 samples, corresponding to day 3 cleavage stage (n=10), and day 5 blastocyst stage (n=10), spent embryo cultures media were processed. Embryo free incubation media as G1 (from day 0 to day3) and G2 (from day 3 to day 5) as negative controls were also passed along the extraction procedure. cftDNA from individual embryos, the blank media controls and human genomic DNA as positive control were amplified with LightCycler PCR 2.0 system. The PCR amplification was performed with LightCycler FastStart DNA Master Plus SYBR Green I. (Cat.N.: 03515869001).

Ingredients: SYBRgreen PCR mix 5x: 4 µL; H₂O: 9 µL; Fwd/Rev primers (see Table 1): 1.0 µL (10x); DNA template: 5 µL. The total reaction volume was 20 µL. Qualitative real time PCR was performed under the following conditions: initial step of 95°C for 10 minutes, 45 cycles of 95°C for 10s, 55°C for 10 s, and 72°C for 10s with 20 °C/s ramp rate; melting curve analysis was performed at of 95°C for 0s, 65°C for 1min, and 95°C for 0s with 0,1 °C/s ramp rate. Results were analysed by LightCycler 4.05 software version.

### Results

Based on our results the tested three genes as: mdr1, prothrombin and leiden were detected in every samples (Fig. 3). The average Ct values in the embryonic spent medium samples occurred as followings: 33 for mdr1 gene, 35 for prothrombin gene and 35 for leiden gene. The average Ct values in human control sample occurred as followings: 20 for mdr1 gene, 20 for leiden gene and 22 for prothrombin gene. In order to prove specificity of the test we have done melting curve analysis (Fig.4.) of the tested genes. Our results indicate that melting temperatures detected in the human control DNA and in the embryonic spent medium samples are correspond to each other. Melting temperatures occurred as followings: 88°C for mdr1 gene, 85°C for prothrombin gene and 82°C for leiden gene.

### Reference Example 2 - Short tandem repeat (STR) profiling

### Sample selection

Based on a well standardized DNA extraction method, a total of 50 samples, corresponding to day 3 cleavage stage (n=24), and day 5 blastocyst stage (n=26), spent embryo cultures media were processed.

### Multiplex PCR amplification

Multiplex PCR amplification of the European standard set (EES) of 15 STR loci and fluorescent dye technology in the automated detection and analysis of DNA profile were performed according to the manufacturer's instructions with minor modification. Briefly: based on quantification of culture media cftDNA content, total amounts of template DNA was setting up in the range of 0.2-0.5 ng for each PCR reaction in a reduced final volume of 12.5 µl. Otherwise standard PCR cycling protocol was applied on a T100 thermal cycler (Bio-Rad Laboratories, Inc., Hercules, CA, USA) following the Investigator ESSplex Kit guidelines (Qiagen, Hilden, Germany) as recommended by the manufacturer.

### Capillary Electrophoresis

The ESSplex amplified STR samples were prepared for electrophoresis by combining highly deionized formamide, GeneScan™ 500 LIZ™ size standard (Applied Biosystems, Foster City, CA, USA) with 1.5 µl of amplified product. After denaturation (95° C for 3 min) and snap-cooling (3 min) the prepared samples were injected sequentially onto an ABI Prism 310 Genetic Analyzer (Applied Biosystems) using POP-4 polymer and 47 cm capillary at 15 kV for 10 seconds. The collected raw data were stored as .fsa files and analyzed using GeneMapper ID software version 3.2 with a peak amplitude threshold of 100 RFU and the default stutter filter settings 20%.

### Statistical analyses

Statistical analyses such as Pearson's correlation coefficient (r), Kendall's tau-b correlation coefficient (τ), R-squared (R²) and probability (p-value) calculations were performed using IBM SPSS Statistics for Windows, Version 20.0 (IBM, Armonk, NY, USA). The strength of the correlations was evaluated accordingly: no or weak correlation (0 < r < ± 0.3); moderate correlation (± 0.3 < *r* < ± 0.7); and strong correlation (*r* > ± 0.7). Data were considered statistically significant at *p*-value <0.05.

### Results

### Detection and quantification of cftDNA of spent embryo culture media

Total of 50 samples, corresponding to day 3 cleavage stage (n=24), and day 5 blastocyst stage (n=26), spent embryo cultures media were processed. Regardless of the length of embryo incubation time and actual embryo morphology assessment (Table 2), each samples revealed measurable amount of cftDNA ranging from 44.1 to 253.6 ng/µl (average 68.75 ng/µl).

**Table 2. Morphological assessment of day-3 and day-5 embryos.**

| **Embryo properties** | | | | | |
|---|---|---|---|---|---|
| **Sample ID** | **Days** | **FR** | **DE** | **ICM** | **TE** |
| 188/3, 416/7, 416/6, 121/4, 412/3, 184/1, 376/2, 138/5, 113/3, 251/3, 201/2, 474/8, 412/1, 430/3, 26/4 | 3 | 1 (good) | | | |
| 230/3, 416/8, 192/4, 236/2 , 245/2, 379/1, 229/1, 431/3 | 3 | 2 (fair) | | | |
| 364/3 | 3 | 3 (poor) | | | |
| 41/2, 33/1, 33/2, 38/2, 31/1, 41/3, 23/1, 37/2, 31/2 | 5 | | 1 (early) | 2 (fair) | 1 (fair) |
| 23/2 | 5 | | 1 (early) | 1 (good) | 2 (fair) |
| 41/1 | 5 | | 1 (early) | 2 (fair) | 3 (poor) |
| 31/3 | 5 | | 1 (early) | 3 (poor) | 3 (poor) |
| 38/1, 19/1, 19/2 | 5 | | 2 (blast.) | 1 (good) | 1 (good) |
| 22/1, 46/1 | 5 | | 2 (blast.) | 2 (fair) | 1 (good) |
| 34/1, 47/1, 29/2, 67/2, 58/1, 29/3 | 5 | | 2 (blast.) | 2 (fair) | 2 (fair) |
| 46/2 | 5 | | 2 (blast.) | 3 (poor) | 2 (fair) |
| 37/1 | 5 | | 2 (blast.) | 1 (good) | 3 (poor) |
| 47/2 | 5 | | 2 (blast.) | 2 (fair) | 3 (poor) |

In order to improve measurement accuracy of NanoDrop readings and estimate size distribution of cftDNA, samples were re-assayed on a microfluidics-based Agilent Bioanalyzer platform, and visualized by pseudo-gel images as well as traditional gel electrophoresis. Overall, the resulting data clearly indicated that UV absorbance measurement at 260 nm tend to massively overestimate (approximately ten times) prevalence of cftDNA. However, pairwise comparison of cftDNA concentrations revealed good concordance between the two techniques (Kendall's τ was 0.895, *p* = 0.000). Based on the clarified cftDNA levels, statistically significant (*p* = 0.038) linear relationship (*r* = 0.410) was found between days of incubation (day 3 and day 5 after ICSI) and amounts of cftDNA recovered from the spent embryo culture media.

### Reference Example 3 - STRs profiling of cftDNA recovered from spent embryo culture media

Based on quality-controlled commercial kit (see Materials and Methods section) multiplex PCR amplification of 15 individual STRs, located on 13 different autosomes were evaluated. Locus name, chromosomal location, repeat motif of the reference allele, average size of the amplified products (bp) and proportion (%) of positive PCR amplifications are summarized in Table 3.

**Table 3. Locus-specific information for the STR markers.**

| **Locus** | **Location** | **Repeat motif** | **Size (bp)** | **% efficiency** |
|---|---|---|---|---|
| D1S1656 | 1q42 | [TAGA]₁₆ [TGA] [TAGA] [TAGG]₁ [TG]₅ | 180.684 | 78.72 |
| D2S441 | 2p14 | [TCTA]₁₂ | 100.043 | 72.34 |
| D2S1338 | 2q35 | [TGCC]6 [TTCC]₁₁ | 409.632 | 59.57 |
| D3S1358 | 3p25.3 | TCTA [TCTG]₂ [TCTA]₁₅ | 177.938 | 78.72 |
| D8S1179 | 8q23.1-23.2 | [TCTA]₁₂ | 312.081 | 57.44 |
| D10S1248 | 10q26.3 | [GGAA]₁₃ | 109.965 | 78.72 |
| D12S391 | 12p13.2 | [AGAT]₅ GAT [AGAT]₇ [AGAC]₆ AGAT | 251.384 | 80.85 |
| D16S539 | 16q24.1 | [GATA]₁₁ | 114.356 | 76.59 |
| D18S51 | 18q21.3 | [AGAA]₁₃ | 173.022 | 78.72 |
| D19S433 | 19q12 | AAGG [AAAG] AAGG | 253.693 | 82.9 |
| D21S11 | 21q21.1 | TAGG [AAGG]₁₁ | 351.940 | 48.93 |
| D22S1045 | 22q12.3 | [ATT]₁₄ ACT [ATT]₂ | 174.056 | 80.85 |
| FGA | 4q28.2 | [TTTC]₃ TTTTTTCT | 329.685 | 65.95 |
| TH01 | 11p15.5 | [CTTT]₁₃ CTCC [TTCC]₂ | 95.088 | 78.72 |
| vWA | 12p13.31 | TCTA [TCTG]₄ [TCTA]₁₃ | 267.865 | 78.72 |

To acquire valuable information about accuracy and characteristics of cftDNA profiling, additional factors such as size and complexity of core repeat motifs, mutation rate of loci and length of the amplicons, which can affect STRs amplification efficiency were also taking into consideration. All but one of the 15 STR markers employed in the present study were tetranucleotide with mostly simple or compound repeat structures (Table 3). The mutation rates of the individual locus vary between 0.01 % and 0.22 % (NIST, STRBase; http://www.cstl.nist.gov/biotech/strbase), nevertheless all of them belong to the group of low mutation rates loci (< 0.3 %). To evaluate the overall performance of the amplification and potential stochastic fluctuations in the typing procedures, PCR product with DNA template purified from healthy Caucasian volunteers as positive controls were assayed. The STR fingerprint generated from the positive control samples revealed full profiles and all alleles were clearly visible above the calling threshold (100 RFU) (Fig. 5A). These results demonstrated that, certain trait factors of autosomal STR loci i.e. repeat classes (tri- or tetranucleotide), complexity of core repeat motifs (simple, compound, or complex), and differences in mutation rate of loci if that is less than 0.3 % do not appear to have strong primary effect on the effectiveness of STR profiling when unlimited amounts of high quality DNA templates were available.

After method validation and quality control procedures, fifty spent embryo cultures media were analyzed by multiplex STR assay. Most of these samples had failed to generate a complete profile using standard PCR conditions. Out of 50 recovered cftDNA samples 32 revealed partial, 15 full STR profiles, while the remaining three samples did not provide appreciable results. Representative micrographs of day 3 (cleavage stage) and day 5 (blastocyst stage) embryos with their electropherograms generated from the corresponding spent embryo cultures media are shown in Fig. 5B and 5C.

In order to provide a thorough and detailed insight into STR fingerprint of individual spent embryo culture media, sizes of locus specific amplicons was computed against amplification efficiency. In line with the theoretical expectations negative correlation was detected between the length of amplicons and they amplification efficiency (Pearson correlation: r = -0.683, regression value *R²* = 0.467, is significant at the *p* = 0.01 level) (Fig. 6A). The collective data indicated that shorter amplicons were less sensitive to degradation and signal losses for larger sized PCR products were more characteristic. However it is also interesting to note that some loci e.g., D21S11, D8S1179, D2S441 and D2S1338 are reside at chromosome locations 21q21.1, 8q23.1-23.22q35, 2q35 and 2p14 respectively, revealed greater distance (deviation) from data point to the trend line, infer that size of loci could not explain all the variation in amplification success (Fig. 6B).

Even though several lines of evidence have substantiated a link between degrees of cytoplasmic fragmentation and developmental competence of human preimplantation embryos, little is known about whether cftDNA degradation is related to specific embryo parameters and/or their implantation potential. To investigate predictive capability of cftDNA profiling, first morphological appearance of cellular fragmentation was graded according to a three-category scoring scheme (good < 10%, fair 10-25%, poor > 25%), (Table 2), and then cftDNA damage was estimated qualitatively by non-invasive STR genotyping. It is generally accepted that quality of template DNA plays an important role in allelic drop-out allowing us to determine cftDNA degradation rather than merely focusing on the amount of cftDNA present in the samples. The extent of cftDNA degradation was expressed in terms of DNA fragmentation index (DFI), which is the ratio of the number of "silent" loci to that can be potentially amplifiable via multiplex PCR. Surprisingly the calculated correlation coefficient (Pearson's r = 0.145) between fragmentation rates of day 3 embryos and cftDNA fragmentation index of the corresponding spent embryo culture media did not support our research hypothesis that the two parameters are directly proportional. Similarly, no correlation was found between DFI of day 3 embryos and pregnancy outcome, nevertheless degree of cytoplasmic fragmentation was inversely correlated with prevalence of live births (r = -0.205), although without reach statistical significance. Despite the fact that DFI was not significantly correlated with grading of single morphological features such as inner cell mass (ICM) and trophectoderm (TE) formation of the blastocyst, clear differences were observed between the median of cftDNA fragmentation of day 3 and day 5 embryos (Fig. 7).

STR markers provide considerable advantages over previously used methods for DNA fingerprinting including the ability to obtain results from small amounts and degraded DNA samples. In good agreement with the theoretical expectation, average success rate of STR amplification was 94.1% (47 evaluable electropherogram of PCR product out of 50 samples).

The high frequency generation of successful STR profiles clearly demonstrated that the extracted cftDNA compliance with the desired standards of quantity and quality for multiplex STR-assay and can be used for whole genome sequencing.

Our data emphasize the belief that introduction of cftDNA fragmentation assays to the non-invasive methodology of ART can assist to improve current embryo selection methods.

### Example 4 - Full genome sequencing to detect aneuploidy

### Genome-wide NGS detection of aneuploidy

### Wetlab materials and methods

Prior to whole-genome amplification, the concentration of cftDNA was determined by using the Qubit dsDNA HS Assay Kit with the Qubit 2.0 Fluorometer according to the manufacturer's (Life Technologies) instructions. Primers 5N3T and 5N3G consisting of eight nucleotides of random sequence and a common 27-nt tag were annealed up to 11 ng of template DNA and were extended by a polymerase in a 30 µl isothermal strand-displacement reaction in 0.2 mL PCR tubes (ABGene) in Veriti 96 Thermocycler (Life Technologies) as follows:

**Table 4. Preparation of the linear amplification mastermix.**

| **Component** | **Volume** |
|---|---|
| Bst 10x Buffer (NEB) | 3 µL |
| 3.2 µM Primer 5N3T (IDT) | 3 µL |
| 5'-GTGAGTGATGGTTGAGGTAGTGTGGAGNNNNNTTT-3' | |
| 2.5 µM Primer 5N3T (IDT) | 3 µL |
| 5'-GTGAGTGATGGTTGAGGTAGTGTGGAGNNNNNGGG-3' | |
| dNTP Mix 25 mM (Fermentas) | 3 µL |
| AccuGene mol. biol. grade water (Lonza) | 18-x µL |
| DNA template | x µL |
| **Total** | **30 µL** |

Tubes are incubated at 94°C for 3 min, then are immediately quenched on ice for 1 min.

**Table 5. Preparation of the polymerase enzyme mix.**

| **Component** | **Volume** |
|---|---|
| Bst Polymerase Large Fragment 8U/µL (NEB) | 0.31 µL |
| Bst 10x Reaction Buffer (NEB) | 0.029 µL |
| AccuGene mol. biol. grade water (Lonza) | 0.261 µL |
| **Total** | **0.6 µL** |

Then 0.6 µL of the polymerase mix is added into each PCR tubes and the following temperature steps are applied: 45 s at 10°C, 45 s at 20°C, 45 s at 30°C, 45 s at 40°C, 45 s at 50°C, 2 min at 65°C and 20 s at 95°C. The tubes are then quickly quenched on ice for 1 min followed by the addition of the same amount of polymerase. After five cycles of linear amplification by using the temperature steps 45 s at 10°C, 45 s at 20°C, 45 s at 30°C, 45 s at 40°C, 45 s at 50°C, 3 min at 62°C, 20 s at 95°C and 20 s at 58°C, the resulting DNA was amplified further by standard high-fidelity PCR to a level sufficient for next-generation sequencing.

**Table 6. Preparation of the standard amplification master mix.**

| **Component** | **Volume** |
|---|---|
| Linear amplification product | 30 µL |
| Phusion 5x Reaction Buffer (Thermo Scientific) | 10 µL |
| Phusion Hot Start II High-Fidelity DNA Polymerase (Thermo Scientific) | 0.5 µL |
| dNTP mix 10 mM (Fermentas) | 1 µL |
| Primer BioGAT 9.9 µM (IDT) | 3.3 µL |
| 5'-Biotin-GTGAGTGATGGTTGAGGTAGTGTGGAG-3' | |
| AccuGene mol. biol. grade water (Lonza) | 5.2 µL |
| **Total** | **50 µL** |

For standard PCR, the following program was used: 30 s at 98 °C; 18 cycles of 15 s at 98 °C, 20 s at 59 °C and 1 min at 72 °C; and 1 min at 72 °C.

The concentration of the product was determined by using the Qubit dsDNA HS Assay Kit, and 100 ng of Double-stranded DNA product was fragmented by using the NEBNext Fast DNA Fragmentation & Library Preparation Kit (NEB) as follows:

**Table 7. Preparation of DNA fragmentation master mix.**

| **Component** | **Volume** |
|---|---|
| 100 ng of dsDNA product | x µL |
| AccuGene mol. biol. grade water (Lonza) | 17.25-x µL |
| Fragmentation Reaction Buffer (NEB) | 2 µL |
| Fragmentation Master Mix (NEB) | 0.75 µL |
| **Total** | **20 µL** |

After mixing by pipetting, the PCR tubes are incubated for 5 min at 25 °C and then for 10 min at 70 °C. Biotinylated fragments are removed by using Ion PGM Enrichment Beads (Dynabeads MyOne Streptavidin C-1, Life Technologies) according to the following protocol. Magnetic Beads are vortexed for 10 s, then its suitable volume is added to a new 1.5-mL LoBind Tube (Eppendorf) containing ten volume MyOne Beads Wash Solution. After pipetting the solution up and down 10 times, tubes are placed on a DynaMag-2 magnet for 2 minutes, then the supernatant is removed and discarded. Ten volume Wash Solution is added againg and the beads are resuspended by pipetting. 2 µL of the prepared suspension is transferred to each sample in LoBind Tubes, which are shaked at 800 rpm for 30 min. Tubes are placed on a DynaMag-2 magnet for 2 minutes and the supernatant is used for the following adaptor ligation procedure.

**Table 8. Preparation of ligation master mix.**

| **Component** | **Volume** |
|---|---|
| T4 DNA Ligase puffer (NEB) | 4 µL |
| *Bst* DNA polymerase (NEB) | 1 µL |
| Adaptor P1 1.4 µM (KAPA) | 4 µL |
| Barcoded Adaptor 1.4 µM (KAPA) | 4 µL |
| T4 DNA Ligase (NEB) | 4 µL |
| AccuGene mol. biol. grade water (Lonza) | 3 µL |
| Template DNA | 20 µL |
| **Total** | **40 µL** |

Reaction mixtures are pipetted up and down 5 times, transferred to PCR tubes, which are incubated for 15 min at 25 °C and then for 5 min at 65 °C. After addition of 5 µL Stop Buffer, vortexing for 10 s and pulse-spin, the reaction volumes are supplemented with 55 µL of Accugene water.

AgenCourt AMPure XP Kit (Beckman Coulter) is applied to size-select the fragmented and adaptor-ligated DNA with two rounds of binding to the paramagnetic beads followed by wash and elution. The first round selectively removes DNA > 500 bp by the AMPure XP beads, whilst the second round eliminates DNA <200 bp from the supernatant.

Add 130 µL diluted AMPure Reagent, consisting of 20 µL AMPure Beads and 110 µL 1.8 M NaCl-15.6% PEG 6000 (Sigma-Aldrich), to the sample, pipet up and down 5 times to mix the bead suspension with the DNA thoroughly, then pulse-spin and incubate at room temperature 5 minutes. Pulse-spin and place the sample tube in a DynaMag-2 magnetic rack for 5 minutes. Carefully transfer the supernatant to a new 1.5 mL LoBind tube and add 20 µL of Agencourt AMPure XP Reagent beads in the second round. Pipet up and down 5 times to thoroughly mix the bead suspension with the DNA, then pulse-spin and incubate at room temperature 5 minutes. Pulse-spin and place the sample tube in the magnetic rack for 5 minutes or until the solution clears. Remove and discard the supernatant without disturbing the bead pellet. Without removing the tube from the magnet, dispense 200 µL of freshly prepared 80 % ethanol to the sample. Incubate for 30 seconds, turning the tube around four times in the magnet to move the beads around. After the solution clears, remove and discard the supernatant without disturbing the pellet, and repeat this step for a second wash. To remove residual ethanol, spin the tube (10 s, 6000 rpm), place it back in the magnetic rack, and carefully remove any remaining supernatant with a 20-µL pipettor without disturbing the pellet. Keeping the tube on the magnet, air-dry the beads at room temperature for 1 min. Remove the tube from the magnet, and add 46 µL of AccuGene water to the sample. Pipet the mixture up and down 5 times, place the tube in the magnetic rack for 2 min. After the solution clears, transfer the supernatant containing the eluted DNA to a new 0.2 mL PCR tube without disturbing the pellet.

**Table 9. Preparation of library amplification master mix.**

| **Component** | **Volume** |
|---|---|
| Adaptor-ligated, size-selected DNA | 46 µL |
| Ion Torrent Primer Mix (NEB) | 4 µL |
| NEBNext High-Fidelity 2X PCR Master Mix (NEB) | 50 µL |
| **Total** | **100 µL** |

After pipetting up and down the mixture 5 times, the following PCR program was used: 30 s at 98 °C; 8 cycles of 10 s at 98 °C, 30 s at 58 °C and 30 s at 72 °C; and 5 min at 72 °C.

Primers are removed by using 20 µL of AgenCourt AMPure XP Reagent diluted with 130 µL of 1.8 M NaCl-15.6% PEG 6000 in a single-round setup. After 80 % ethanol wash two times, library DNA was eluted with 20 µL AccuGene water.

Prepared libraries were qualified and quantified with Bioanalyzer 2100 (Agilent). After emulsion PCR and target enrichment (Ion PGM Template OT2 200 Kit), unidirectional sequencing of the pooled library with ca. 0.01x read coverage was performed on an Ion Torrent PGM system using the Ion 318 Chip Kit v2 and the Ion PGM Sequencing 200 Kit v2 according to the Life Technologies' protocol.

### Aneuploidy analysis process - bioinformatics and statistical methods

During sequence processing the first step is the filtering of raw sequence data based on an average quality scores (Q). The Q value is calculated by Ion PGM's quality score system which uses a set of predictors whose values are correlated with the probability of a base miscall. Second step is the trimming, where primer sequences (which are used in library preparation) are removed. After the trimming, the mapping program Bowtie2 (with Burrows-Wheeler transform) is applied to map reads to the human reference genome (hg19). Bowtie2 is a fast and memory-efficient tool for aligning short sequencing reads to long reference sequences.

After the mapping step, somatic chromosomes' absolute read numbers are used to calculate read percentage values , which are normalized to the nominal length of the given chromosome. Hence the bigger the autosomes, the higher the corresponding read numbers. First the reference (baseline) read percentage must be constructed based on DNA samples isolated from peripheral blood of healthy volunteers. Real chromosome ratio values refer as normalized read percentage value from the IVF case divided by the reference read percentage.

Main descriptive statistics (mean and standard deviation) of chromosome ratios are used to calculate z-score values. The involvement of z-score values ensures the application of statistical tests (z-test) for exact comparison of different cases (e.g. test case vs. baseline). Moreover it standardizes chromosome ratios on the same scale, therefore, the visual comparison of test and control cases are also very effective (*e.g.* an additional z-score diagram). For statistical evaluation and graphics, the R statistical environment was used.

### Results

Aneuploidy detection method has been validated on samples originating from healthy males, females, individuals with Down and Kleinefelter syndromes, and from pseudodiploid REH2 and hyperdiploid MHH-CALL2 kariotyped cell lines. Our results revealed not only the reduced amplification bias as an outcome of the linear amplification, but equal distribution of sequenced reads across the whole genome.

Based on our findings the chromosome ratios of each successful pregnancies are between 0.7 and 1.35. Interestingly, all of the tested morphologically non-viable embryos are between these artificial thresholds, however we could detect significant aneuplody events in either successfully or unsuccessfully implantable 3 and 5-day embryos (see Fig. 8.; ratios >1.35). Both pregnancy started from embryos having chromosome ratios higher than 1.35 have lead to spontaneous abortion. All dots laying outside this threshold correspond to non-viable, unsuccessfully implantable or aborted embryos.

It can be concluded that NGS-based aneuplody detection method evaluated by using statistical z-tests appears to efficiently forecast the lethal genetic aneuploidies, and in line with it, the yield of IVF live births can likely be increased forward, however other factors may play major role in the success rate of implantation process itself too.

### References

Arce JC, Nyboe Andersen A, Collins J. Resolving methodological and clinical issues in the design of efficacy trials in assisted reproductive technologies: a mini-review. Human Reproduction 2005; 20:1757-1771.
Balaban B, Brison D, Calderon G, Catt J, Conaghan J, Cowan L, Ebner T, Gardner D, Hardarson T, Lundin K, et al. The Istanbul consensus workshop on embryo assessment: proceedings of an expert meeting. Human Reproduction 2011; 26(6):1270-1283.
Bódis J, Várnagy A, Sulyok E, Kovács GL, Martens-Lobenhoffer J, Bode-Böger SM. Negative association of L-arginine methylation products with oocyte numbers. Human Reproduction 2010; 25(12):3095-3100.
Devroey P, VanSterrteghem A. A review of ten years experience of ICSI. Human Reproduction Update 2004; 10:19-28.
Fujimoto VY, Browne RW, Bloom MS, Sakkas D, Alikani M. Pathogenesis, developmental consequences, and clinical correlations of human embryo fragmentation. Fertility and Sterility 2011; 95:1197-1204.
Gnoth C, Godehardt E, Frank-Herrmann P, Friol K, Tigges J, Freundl G. Definition and prevalence of subfertility and infertility. Human Reproduction 2005; 20(5):1144-1147.
Habbema JD, Eijkemans MJ, Nargund G, Beets G, Leridon H, Te Velde ER. The effect of in vitro fertilization on birth rates in western countries. Human Reproduction 2009; 24(6):1414-1419.
Handyside, A.H., Kontogianni, E.H., Hardy, K. Pregnancies from biopsied human preimplantation embryos sexed by Y-specific DNA. Nature 1990;, 344: 768-770.
Hansen M, Bower C, Milne E, de Klerk N, Kurinczuk JJ. Assisted reproductive technologies and the risk of birth defects - a systematic review. Human Reproduction 2005; 20(2):328-338.
Holdenrieder S, Stieber P. Clinical use of circulating nucleosomes. Critical Reviews in Clinical Laboratory Sciences 2009; 46(1):1-24.
Levy B, Norwitz E. Non-invasive prenatal aneuploidy testing: technologies and clinical implication. MLO Med Lab Obs 2013; 45 (6): 8, 10, 12.
Machtinger R, Racowsky C. Morphological systems of human embryo assessment and clinical evidence. Reproductive BioMedicine Online 2013; 26(3):210-221.
Stigliani S, Anserini P, Venturini PL, Scaruffi P. Mitochondrial DNA content in embryo culture medium is significantly associated with human embryo fragmentation. Human Reproduction 2013; 28(10):2652-2660.
Vernon M, Stern JE, Ball GD, Wininger D, Mayer J, Racowsky C. Utility of the national embryo morphology data collection by the Society for Assisted Reproductive Technologies (SART): correlation between day-3 morphology grade and live-birth outcome. Fertility and Sterility 2011; 95:2761-2763.
Wang J, Sauer MV. In vitro fertilization (IVF): a review of 3 decades of clinical innovation and technological advancement. Therapeutics and Clinical Risk Management 2006; 2(4): 355-364.

## Claims

1. *In vitro* method for non-invasive preimplantation assessment of an embryo, comprising
a) providing a sample taken from the *in vitro* culture medium of the embryo on the 3^{rd} day of incubation;
b) submitting the sample to nucleic acid amplification with primers specific to a sequence of interest indicative of a genetic deficiency;
c) detecting the amplified nucleic acid by PCR,
wherein the presence of the nucleic acid amplified from the culture medium suggests the presence of the corresponding sequence in the embryo cultured in said medium, thereby enabling the preimplantation assessment of the embryo with respect of the sequence of interest, and
wherein the embryo is assessed as having a genetic deficiency if the presence of the sequence of interest is detected in the *in vitro* culture medium of the embryo.

2. The method according to claim 1, wherein the PCR is real time PCR.

3. The method according to claim 1 or 2, wherein the detection includes detecting fluorescent labels.

4. The method according to any one of claims 1 to 3, wherein the sequence of interest is a gene and/or an allele thereof and/or a variant thereof carrying a specific mutation.

5. The method according to claim 4, wherein the gene is located on the Y chromosome.

6. The method according to claim 4 or 5, wherein the gene is known to be involved in developmental disorders.

7. The method according to any one of claims 1 to 3, wherein the method further involves carrying out linear amplification-based full genome sequencing, wherein the presence of aneuploidy is indicative of an embryo having a limited chance for resulting in successful pregnancy.

8. The method according to claim 7, wherein aneuploidy is evaluated by z-tests.

## Patentansprüche

1. *In vitro* Verfahren zur nicht-invasiven Präimplantationsbeurteilung eines Embryos, enthaltend
a) die Bereitstellung einer Probe aus dem *in vitro* Kulturmedium des Embryos am dritten Tag der Inkubation;
b) das Durchführen einer Nukleinsäureamplifikation an der Probe mit Primeren, welche für eine auf einen genetischen Mangel hinweisende Sequenz von Interesse spezifisch sind;
c) das Nachweisen der amplifizierten Nukleinsäure durch PCR,
wobei die Anwesenheit der aus dem Kulturmedium amplifizierten Nukleinsäure auf die Anwesenheit der entsprechenden Sequenz in dem im Kulturmedium kultivierten Embryo hindeutet, wodurch die Präimplantationsbeurteilung des Embryos im Hinblick auf die Sequenz von Interesse ermöglicht wird, und wobei der Embryo als genetisch mangelbehaftet eingestuft wird, wenn die Anwesenheit der Sequenz von Interesse im *in vitro* Kulturmedium des Embryos nachgewiesen wird.

2. Das Verfahren nach Anspruch 1, wobei das PCR ein Echtzeit-PCR ist.

3. Das Verfahren nach Anspruch 1 oder 2, wobei der Nachweis den Nachweis von fluoreszierenden Markierungen einschließt.

4. Das Verfahren nach einem der Ansprüche 1 bis 3, wobei die Sequenz von Interesse ein Gen und/oder ein Allel davon und/oder eine Variante davon ist, welche eine spezifische Mutation tragen.

5. Das Verfahren nach Anspruch 4, wobei das Gen am Y Chromosom lokalisiert ist.

6. Das Verfahren nach Anspruch 4 oder 5, wobei das Gen bekanntermaßen an Entwicklungsstörungen beteiligt ist.

7. Das Verfahren nach einem der Ansprüche 1 bis 3, wobei das Verfahren weiterhin das Durchführen einer auf linearer Amplifikation basierenden vollständigen Genomsequenzierung enthält, wobei das Vorkommen von Aneuploidie ein Hinweis auf einen Embryo ist, der eine begrenzte Chance hat, zu einer erfolgreichen Schwangerschaft zu führen.

8. Das Verfahren nach Anspruch 7, wobei die Aneuploidie durch Z-Tests beurteilt wird.

## Revendications

1. Méthode *in vitro* pour l'évaluation préimplantatoire non invasive d'un embryon, comprenant
a) fournir un échantillon prélevé sur le milieu de culture *in vitro* de l'embryon le 3^{ème} jour d'incubation;
b) soumettre l'échantillon à une amplification d'acide nucléique avec des amorces spécifiques à une séquence d'intérêt indiquant une déficience génétique;
c) détecter l'acide nucléique amplifié par PCR,
où la présence de l'acide nucléique amplifié à partir du milieu de culture suggère la présence de la séquence correspondante dans l'embryon cultivé dans ledit milieu, permettant ainsi l'évaluation préimplantatoire de l'embryon par rapport à la séquence d'intérêt, et
où l'embryon est évalué comme ayant une déficience génétique si la présence de la séquence d'intérêt est détectée dans le milieu de culture *in vitro* de l'embryon.

2. La méthode selon la revendication 1, où la PCR est une PCR en temps réel.

3. La méthode selon la revendication 1 ou 2, où la détection comprend la détection des étiquettes fluorescents.

4. La méthode selon l'une quelconque des revendications 1 à 3, où la séquence d'intérêt est un gène et/ou un allèle de celui-ci et/ou une variante de celui-ci portant une mutation spécifique.

5. La méthode selon la revendication 4, où le gène est situé sur le chromosome Y.

6. La méthode selon la revendication 4 ou 5, où le gène est connu pour être impliqué dans des troubles du développement.

7. La méthode selon l'une quelconque des revendications 1 à 3, où la méthode implique en outre la réalisation d'un séquençage complet du génome basé sur une amplification linéaire, où la présence d'aneuploïdie indique qu'un embryon a une chance limitée de conduire à une grossesse réussie.

8. La méthode selon la revendication 1, où l'aneuploïdie est évaluée par des tests z.
